# EUROPÄISCHE PATENTSCHRIFT

(11) **EP 1 214 914 B1**
(45) Veröffentlichungstag und Bekanntmachung des Hinweises auf die Patenterteilung: **19.05.2004**
(21) Anmeldenummer: 01890340.1
(22) Anmeldetag: 17.12.2001
(51) Int. Cl.: A61B 17/86, A61B 17/72

(54) **Verriegelungsschraube für Implantate**
Locking screw for implants
Vis de blocage pour implants

(30) Priorität: 18.12.2000 AT 2000923 U
(43) Veröffentlichungstag der Anmeldung: 19.06.2002
(73) Patentinhaber: Bernsteiner, Helga Mag.,, Graz 8047 (AT); Ruprechter, Eva, 8047 Graz (AT)
(72) Erfinder: Bernsteiner, Herbert, (AT); Ruprechter, Eva, 8047 Graz (AT); Hertz, Harald Primarius, 1180 Wien (AT)
(74) Vertreter: Haffner, Thomas M., Dr.

(56) Entgegenhaltungen:
- EP-A- 0 856 293
- WO-A-00/40164
- FR-A- 2 766 355
- US-A- 5 019 079

## Beschreibung

Die Erfindung bezieht sich auf eine Verriegelungsschraube für Implantate, wie z.B. Marknägel, insbesondere Tibiamarknägel, welche eine Durchbrechung oder Ausnehmung des Implantates durchsetzend in eine Knochenwand einschraubbar ist, wobei die Verriegelungsschraube in ihrem mittleren die Durchbrechung oder Ausnehmung des Implantates durchsetzenden Bereich als gewindeloser Bolzen ausgebildet ist, dessen Außendurchmesser der Kontur der Durchbrechung oder der Ausnehmung im Implantat im wesentlichen entspricht und größer oder gleich dem Außendurchmesser des Gewindes am freien Ende der Verriegelungsschraube ist und wobei die Verriegelungsschraube zwischen dem mittleren Bereich und dem Schraubenkopf ein weiteres Gewinde mit dem Durchmesser des mittleren Bereiches übersteigendem Außendurchmesser aufweist.

Verriegelungsschrauben dienen dazu, Implantate in ihrer gewünschten Position zu halten. Beispielsweise im Zusammenhang mit Marknägeln und insbesondere Tibiamarknägel dienen derartige Verriegelungsschrauben der Festlegung des Marknagels im proximalen und im distalen Bereich, wobei nach der Festlegung Belastungen des Knochens als Querkräfte von den Verriegelungsschrauben aufgenommen werden müssen. Für eine wirkungsvolle Verriegelung werden derartige Schrauben durch entsprechende Durchbrechungen bzw. Bohrungen im proximalen bzw. distalen Bereich eines Marknagels hindurchgesteckt und mit dem Knochen verschraubt. Eine wirksame Festlegung und Aufnahme der Kräfte kann hiebei nur dann gewährleistet sein, wenn der Durchmesser der Schraube mit der lichten Weite der Durchbrechung übereinstimmt, was insbesondere für die Gewährleistung der erforderlichen Rotationsstabilität und für die Entlastung des Knochens bei Belastung von wesentlicher Bedeutung ist. Verriegelungsschrauben, mit welchen derartige Implantate, wie beispielsweise Tibiamarknägel, festgelegt werden, weisen üblicherweise über ihre gesamte axiale Länge ein durchgehendes Gewinde auf, sodaß der Gewindeaußendurchmesser bzw. Schraubenaußendurchmesser im Bereich der Durchbrechung des Implantates und insbesondere des Tibiamarknagels an der Innenwand der entsprechenden Durchbrechung bzw. Bohrung des Implantates anliegt, um die Kräfte als Scherkräfte aufnehmen zu können. Die Ausbildung eines Gewindes im Bereich des Eingriffes der Verriegelungsschrauben in die entsprechende Ausnehmung des Implantates führt aber zu einer Schwächung der Verriegelungsschrauben und zu einer Kerbbelastung, welche hohe Ansprüche an die Festigkeit der Verriegelunggschraube stellt. Derartige Verriegelungsschrauben müssen daher in der Regel überdimensioniert werden, um die gewünschten Querkräfte sicher aufnehmen zu können und es muß daher in aller Regel auch ein entsprechend überdimensioniertes Gewinde in den Knochen eingeschraubt werden. Bedingt durch die erforderlichen Festigkeitseigenschaften und insbesondere die geforderte Aufnahme der Querkräfte durch die Verriegelungsschrauben werden zu allem Überfluß beispielsweise bei der Festlegung und Verriegelung von Tibiamarknägel im proximalen und im distalen Bereich Schrauben unterschiedlicher Durchmesser eingesetzt, um diesen Belastungen gerecht werden zu können.

Im Zusammenhang mit sogenannten Spannschrauben, mit welchen es gelingt, Knochensplitter unmittelbar miteinander zu verbinden und gegeneinander zu spannen, ist es bereits bekannt derartige Spannschrauben mit einem mittleren gewindelosen Teilbereich auszustatten. Dieser mittlere gewindelose Teilbereich, wie er beispielsweise den Ausbildungen, wie sie in der EP 695 537 A1 oder EP 856 293 A1 beschrieben und gezeigt sind, zu entnehmen ist, dient aber in erster Linie der Lösung fertigungstechnischer Probleme, da eine derartige Spannschraube über ihre axiale Länge Teilbereiche mit unterschiedlicher Gewindesteigung aufweisen muß, um ein Spannen von Knochenteilen gegeneinander zu ermöglichen, und die Herstellung derartiger unterschiedlicher Gewindesteigung am leichtesten unter Zwischenschaltung eines gewindelosen Abschnittes möglich ist. Die zu diesem Zwecke getroffene Ausbildung sieht daher immer vor, daß der gewindelose Abschnitt im wesentlichen dem Kerndurchmesser eines der beiden Gewinde entspricht, sodaß derartige Schrauben als Verriegelungsschrauben nicht geeignet sind, da der gewindelose Abschnitt auf geringerem Durchmesser als der Schraubendurchmesser abgesetzt ist. Nach dem Durchstecken einer derartigen Schraube durch eine entsprechende Durchbrechung eines Implantates würde hier immer ein Spiel im Inneren der Durchbrechung verbleiben, welches die gewünschte Verriegelung verhindert.

Aus der US-A 5 019 079 (Basis für den Oberbegriff des Anspruchs 1.) ist eine Verriegelungsschraube der eingangs genannten Art bekanntgeworden, bei welcher allerdings entsprechend vorgebohrte Öffnungen vorhanden sein müssen, um ein sicheres Einschrauben zu ermöglichen. Insbesondere bei Verwendung derartiger Verriegelungsschrauben für Tibiamarknägel ist es in der Folge erforderlich, daß dem Kopf bzw. dem Betätigungswerkzeug der Schraube abgewandte Ende in eine Innenwand eines Hohlknochens einzuschrauben, wobei bedingt durch anatomische Gegebenheiten in manchen Fällen hier bereits vor dem Zeitpunkt, zu welchem das dem Werkzeug bzw. dem Kopf abgewandte Ende in die Innenwand eingeschraubt werden kann, das größere Gewinde in die Außenwand des Knochens eingeschraubt werden muß. In diesen Fällen wird dieser Einschraubvorgang nicht durch die Spannkräfte des endständigen Gewindes unterstützt und es hat sich gezeigt, daß in Fällen, in welchen das dem Kopf abgewandte Ende noch nicht in die Innenwand eines Knochens eingreift, das weitere Einschrauben derartiger Schrauben nicht ohne weiteres gelingt.

Die Erfindung zielt nun darauf ab, die Einschraubkräfte bei Verwendung derartiger Verriegelungsschrauben wesentlich zu verringern und insbesondere sicherzustellen, daß auch dann, wenn lediglich das zweite Gewinde, welches zwischen dem gewindelosen Bolzen im mittleren Bereich und dem Kopf der Schraube angeordnet ist, ohne Unterstützung durch das erste Gewinde eingeschraubt werden soll, eine sichere Verankerung ermöglicht wird.

Zur Lösung dieser Aufgabe besteht die erfindungsgemäße Verriegelungsschraube im wesentlichen darin, daß der Übergangsbereich zwischen dem mittleren gewindelosen Bereich der Schraube und dem weiteren Gewinde als Gewindeschneider mit Freiräumen für den Abtransport von geschnittenem Material mit einer kegelstumpfförmigen Hüllfläche ausgebildet ist, deren Erzeugende mit der Achse der Schraube einen Winkel von kleiner als 35°, vorzugsweise kleiner als 25°, einschließen. Dadurch, daß nun der Übergangsbereich zwischen dem mittleren gewindelosen Bereich der Schraube und dem weiteren Gewinde als Gewindeschneider ausgebildet ist, wird dann, wenn die Erzeugenden der kegelstumpfförmigen Hüllfläche dieses Gewindeschneiders mit der Achse einen Winkel von kleiner als 35° einschließen, ein sicheres Einschrauben auch dann gewährleistet, wenn das vordere Ende der Verriegelungsschraube noch nicht in Knochenmaterial eingreift und daher nicht zur Unterstützung der Einschraubbewegung zur Verfügung steht. Die Einhaltung eines derartigen spitzen Winkels hat sich als wesentlich für die Praxis herausgestellt und unterscheidet sich grundsätzlich von den bekannten Gewindeenden, welche üblicherweise Winkel von 45° oder größer zwischen den Erzeugenden der Hüllfläche und der Achse der Schraube aufweisen. Insgesamt wird mit einer derartigen Ausbildung das Einschrauben wesentlich erleichtert und gleichzeitig die Gefahr einer überflüssigen Zerstörung von Knochenmaterial wesentlich herabgesetzt.

In besonders bevorzugter Weise ist die erfindungsgemäße Schraube dahingehend weitergebildet, daß der Winkel kleiner 20° und vorzugsweise etwa 15° beträgt.

Auch das dem Kopf abgewandte Ende kann in konventioneller Weise als Gewindeschneider ausgebildet sein, wobei ein besonders schonendes und sicheres Einschrauben dann gelingt, wenn die Ausbildung so getroffen ist, daß der dem Kopf abgewandte Endbereich der Schraube gleichfalls als Gewindeschneider ausgebildet ist, dessen Erzeugende der Hüllfläche mit der Achse einen kleineren Winkel als der zwischen den Erzeugenden und der Achse des in Achsrichtung folgenden Gewindeschneiders aufweisen, wobei vorzugsweise der Winkel im dem Kopf abgewandten Endbereich kleiner als 25° und vorzugsweise etwa 10° beträgt. Insgesamt ergibt sich durch Einhaltung der erfindungsgemäßen Kriterien ein besonders leichtes und sicheres Einschrauben der Verriegelungsschraube. Nach einem Durchstecken der Verriegelungsschraube durch die entsprechende Aufnahmebohrung des Implantates gelingt unmittelbar eine Verschraubung mit der hinterliegenden Kortikalis, wobei gleichzeitig oder aber vor dem Einschrauben in die Gegenkortikalis eine Verschraubung in dem den Schraubenkopf benachbarten Bereich mit entsprechend größerem Durchmesser unmittelbar selbstschneidend vorgenommen werden kann.

Die Verriegelungsschraube kann für spezielle Fälle gleichzeitig auch die Funktion einer Spannschraube erfüllen, sodaß die Fixierung von Splittern gleichzeitig mit der Verriegelung eines Implantates vorgenommen werden kann. Zu diesem Zwecke kann die Ausbildung so getroffen sein, daß die Gewindesteigung des dem Schraubenkopf benachbarten weiteren Gewindes kleiner als die Gewindesteigung des Gewindes am freien Ende der Schraube gewählt ist.

Für den üblichen Einsatz als Verriegelungsschraube mit entsprechend paßgenauem Eingriff in die Durchbrechung des Implantates kann aber die Ausbildung in konventioneller Weise so gebildet sein, daß die Gewindesteigungen der beiden Gewindeabschnitte gleich gewählt sind.

Herstellungstechnisch lassen sich derartige Verriegelungsschrauben besonders einfach dadurch realisieren, daß der Kerndurchmesser des weiteren Gewindes dem Durchmesser des gewindelosen mittleren Bereiches entspricht, wodurch gleichzeitig das Einschrauben der Verriegelungsschraube ohne zusätzliche Bohrung in den Knochen in einem Arbeitsgang ermöglicht wird.

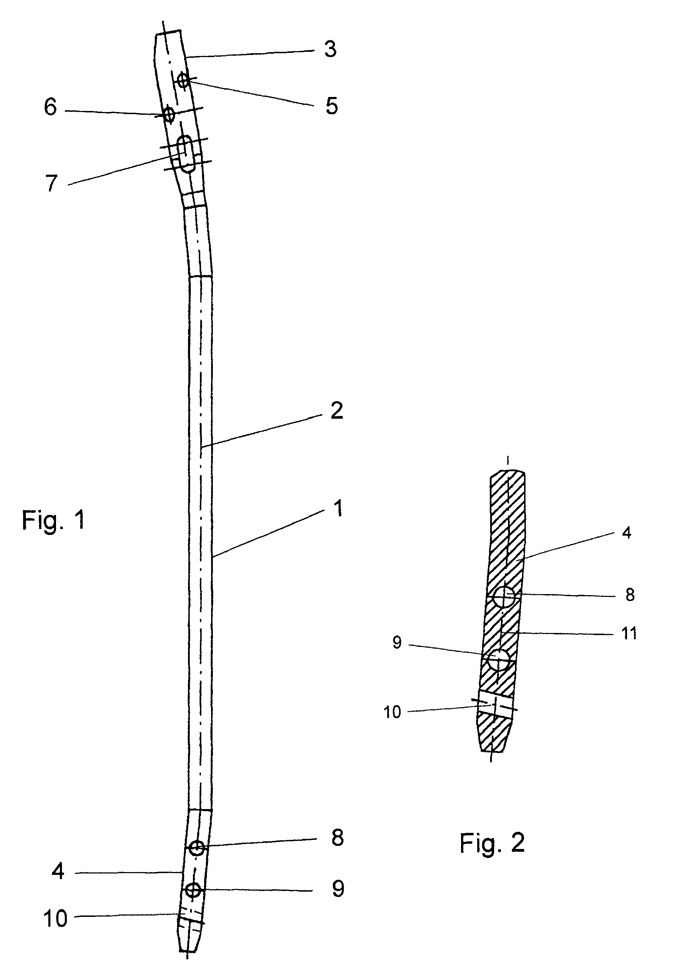

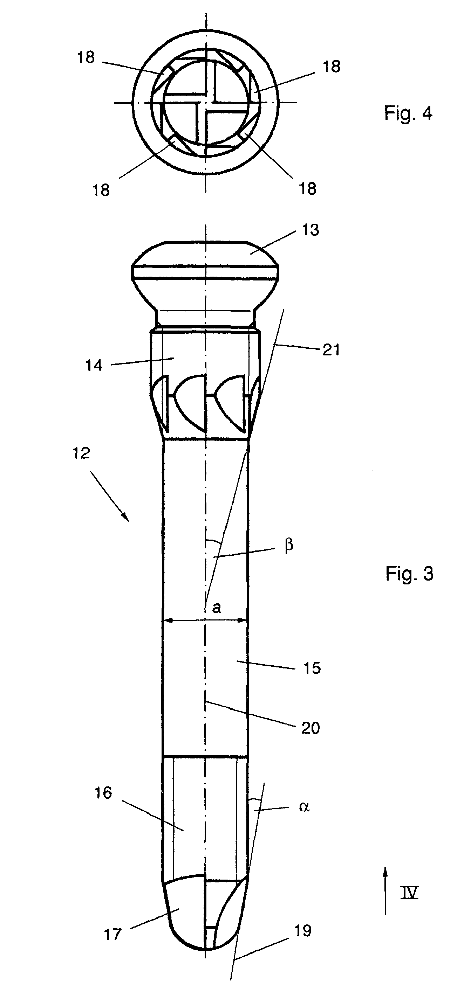

Die Erfindung wird nachfolgend anhand eines in der Zeichnung schematisch dargestellten Ausführungsbeispieles näher erläutert, wobei in der Zeichnung als Beispiel für den Einsatz einer Verriegelungsschraube ein Tibiamarknagel gewählt wird. In der Zeichnung zeigen Fig. 1 eine Seitenansicht eines Tibianagels, Fig. 2 einen Schnitt durch den distalen Endbereich des Tibianagels nach Fig. 1, Fig. 3 eine Ansicht einer erfindungsgemäßen Verriegelungs- oder Spannschraube und Fig. 4 eine Ansicht in Richtung des Pfeiles IV der Fig. 3 auf die Verriegelungsschraube bzw. Spannschraube.

In Fig. 1 ist ein Tibiamarknagel 1 dargestellt, dessen Achse strichpunktiert mit 2 bezeichnet ist. Die Achse des Tibiamarknagels 1 ist hiebei mehrfach gekröpft ausgebildet und in Richtung zum proximalen Ende 3 ebenso wie in Richtung zum distalen Ende 4 gleichsinnig geneigt zur Achse 2 im mittleren Bereich des Tibiamarknagels.

Das proximale Ende weist einander kreuzende Bohrungen 5 und 6 für die Rotationsverriegelung auf. Zusätzlich ist im proximalen Endbereich ein Langloch 7 vorgesehen, über welches eine weitere Verriegelungsschraube eingebracht werden kann, wobei aufgrund der Ausgestaltung der Durchbrechung als Langloch 7 hier eine axiale Bewegung des Tibiamarknagels 1 und damit eine Dynamisierung ermöglicht wird.

Das distale Ende 4 weist wiederum zwei Bohrungen 8 und 9 mit im wesentlichen orthogonal zur Achse 2 verlaufender Bohrungsachse für die Aufnahme von Verriegelungsschrauben auf. Im distalen Endbereich ist in Fig. 1 mit 10 eine nach hinten geneigte Bohrung angedeutet, welche im Schnitt in der Darstellung nach Fig. 2 deutlich ersichtlich ist.

In Fig. 2 ist diese Bohrung 10 unter einem Winkel von etwa 10° zur Achse 11 des distalen Bereiches 4 des Tibianagels geneigt angeordnet, sodaß sich insgesamt eine Gesamtneigung zur Achse 2 im mittleren Bereich des Tibianagels von etwa 15° ergibt. Durch die Bohrungen bzw. Durchbrechungen 5, 6, 7, 8, 9 und 10 können nun konventionelle Verriegelungsschrauben oder aber Spannschrauben geführt werden, um auf diese Weise eine sichere Verankerung des Tibianagels zu gewährleisten und gegebenenfalls zusätzlich Splitter erfolgreich zu fixieren. Eine für die Verwendung mit einem derartigen Tibiamarknagel nach den Fig. 1 und 2 geeignete erfindungsgemäße Spann- bzw. Verriegelungsschraube ist in den Fig. 3 und 4 dargestellt.

In Fig. 3 ist eine Verriegelungsschraube 12 ersichtlich, welche im Anschluß an einen Schraubenkopf 13 einen ersten Gewindeabschnitt 14, einen mittleren gewindelosen Bolzenabschnitt 15 und einen endständigen Gewindeabschnitt 16 aufweist. Am freien Ende der Verriegelungsschraube ist im Anschluß an das Gewinde 16 ein Schneidkopf 17 vorgesehen, bei welchem, wie sich insbesondere aus Fig. 4 ergibt, zwischen Gewindeabschnitten Freiräume 18 für den Abtransport des geschnittenen Materials ausgebildet sind, um ein Einschrauben der Schraube im Knochensplitter bzw. die Kortikalis zu erleichtern.

Der mittlere Abschnitt 15 der Verriegelungsschraube ist hiebei gewindelos ausgebildet und weist einen Außendurchmesser a auf, welcher dem lichten Querschnitt der Durchbrechungen des Tibiamarknagels im wesentlichen entspricht. Um die Durchführung der Verriegelungsschrauben durch die Durchbrechungen zu ermöglichen, ist das endständige Gewinde mit einem Außendurchmesser ausgebildet, welcher maximal diesem Durchmesser a des gewindelosen Bereiches im mittleren Teil entspricht, wobei eine Festlegung im Knochen an der dem freien Ende gegenüberliegenden Seite durch das entsprechend größeren Durchmesser aufweisende Gewinde 14 gewährleistet ist. Wenn die Verriegelungsschraube 12 als Spannschraube eingesetzt werden soll, muß das Gewinde 14 mit geringerer Steigung als das Gewinde 16 ausgebildet sein, sodaß beim Einschrauben ein Anziehen von Knochensplittern in Richtung zum mittleren, gewindelos ausgebildeten Bereich 15 der Schraube 12 erfolgt.

Wie sich nun unmittelbar aus Fig. 3 ergibt, sind die beiden gewindeschneidenden Abschnitte der Gewinde 14 und 16 in Übereinstimmung mit der Erfindung spitzwinkelig ausgebildet. Der Winkel, der von der Erzeugenden 19 des winkelschneidenden Abschnittes des Gewindes 16 mit der Achse 20 der Schraube eingeschlossen wird, ist hiebei mit α bezeichnet und beträgt etwa 10°. Für den Winkel β zwischen den Erzeugenden 21 des gewindeschneidenden Abschnittes des Gewindes 14 und der Achse 20 wurde ein Wert von etwa 15° gewählt.

## Patentansprüche

1. Verriegelungsschraube (12) für Implantate, wie z.B. Marknägel, insbesondere Tibiamarknägel, welche eine Durchbrechung oder Ausnehmung des Implantates durchsetzend in eine Knochenwand einschraubbar ist, wobei die Verriegelungsschraube (12) in ihrem mittleren die Durchbrechung oder Ausnehmung des Implantates durchsetzenden Bereich (15) als gewindeloser Bolzen ausgebildet ist, dessen Außendurchmesser (a) der Kontur der Durchbrechung oder Ausnehmung im Implantat im wesentlichen entspricht und größer oder gleich dem Außendurchmesser des Gewindes (16) am freien Ende der Verriegelungsschraube (12) ist und wobei die Verriegelungsschraube (12) zwischen dem mittleren Bereich (15) und dem Schraubenkopf (13) ein weiteres Gewinde (14) mit dem Durchmesser (a) des mittleren Bereiches (15) übersteigendem Außendurchmesser aufweist, **dadurch gekennzeichnet, daß** der Übergangsbereich zwischen dem mittleren gewindelosen Bereich (15) der Schraube (12) und dem weiteren Gewinde (14) als Gewindeschneider mit Freiräumen für den Abtransport von geschnittenem Material mit einer kegelstumpfförmigen Hüllfläche ausgebildet ist, deren Erzeugende (21) mit der Achse (20) der Schraube (12) einen Winkel (β) von kleiner als 35°, vorzugsweise kleiner als 25°, einschließen.

2. Verriegelungsschraube nach Anspruch 1, **dadurch gekennzeichnet, daß** der Winkel (β) kleiner 20° und vorzugsweise etwa 15° beträgt.

3. Verriegelungsschraube nach Anspruch 1 oder 2, **dadurch gekennzeichnet, daß** der dem Kopf (13) abgewandte Endbereich (17) der Schraube (12) gleichfalls als Gewindeschneider ausgebildet ist, dessen Erzeugende (19) der Hüllfläche mit der Achse (20) einen kleineren Winkel (α) als der zwischen den Erzeugenden (21) und der Achse (20) des in Achsrichtung folgenden Gewindeschneiders aufweisen.

4. Verriegelungsschraube nach Anspruch 3, **dadurch gekennzeichnet, daß** der Winkel (α) im dem Kopf (13) abgewandten Endbereich (17) kleiner als 25° und vorzugsweise etwa 10° beträgt.

5. Verriegelungsschraube nach einem der Ansprüche 1 bis 4, **dadurch gekennzeichnet, daß** der Kerndurchmesser des weiteren Gewindes (14) dem Durchmesser (a) des gewindelosen mittleren Bereiches (15) entspricht.

6. Verriegelungsschraube nach einem der Ansprüche 1 bis 5, **dadurch gekennzeichnet, daß** die Gewindesteigungen der beiden Gewindeabschnitte (14,16) gleich gewählt sind.

7. Verriegelungsschraube nach einem der Ansprüche 1 bis 5, **dadurch gekennzeichnet, daß** die Gewindesteigung des dem Schraubenkopf (13) benachbarten weiteren Gewindes (14) kleiner als die Gewindesteigung des Gewindes (16) am freien Ende der Schraube (17) gewählt ist.

## Claims

1. A locking screw (12) for implants such as, e.g., intramedullary nails and, in particular, tibial intramedullary nails, which is capable of being screwed into a bone wall while passing through a hole or recess of the implant, said locking screw (12), in its central region (15) passing through the hole or recess of the implant, being designed as a threadless pin whose outer diameter (a) substantially corresponds with the contour of the hole or recess provided in the implant and is larger than, or equal to, the outer diameter of the thread (16) provided on the free end of the locking screw (12), and wherein the locking screw (12), between the central region (15) and the screw head (13), comprises a further thread (14) having an outer diameter exceeding the diameter (a) of the central region (15), **characterized in that** the region of transition between the central, threadless region (15) of the screw (12) and the further thread (14) is designed as a thread-cutting means having free spaces for the removal of cut material and a frustoconical enveloping surface whose generatrices (21) enclose an angle (β) smaller than 35°, preferably smaller than 25°, with the axis (20) of the screw (12).

2. A locking screw according to claim 1, **characterized in that** the angle (β) is smaller than 20°, preferably about 15°.

3. A locking screw according to claim 1 or 2, **characterized in that** the end region (17) of the screw (12), that faces away from the head (13) is likewise designed as a thread-cutting means, whose generatrices (19) of the enveloping surface enclose, with the axis (20), an angle (α) smaller than that formed between the generatrices (21) and the axis (20) of the thread-cutting means following in the axial direction.

4. A locking screw according to claim 3, **characterized in that** the angle (α) is smaller than 25°, preferably about 10°, in the end region (17) facing away from the head (13).

5. A locking screw according to any one of claims 1 to 4, **characterized in that** the core diameter of the further thread (14) corresponds to the diameter (a) of the threadless, central region (15).

6. A locking screw according to any one of claims 1 to 5, **characterized in that** the thread pitches of the two threaded sections (14, 16) are chosen to be equal.

7. A locking screw according to any one of claims 1 to 5, **characterized in that** the pitch of the further thread (14) provided in the vicinity of the screw head (13) is smaller than the pitch of the thread (16) provided on the free end of the screw (17).

## Revendications

1. Vis de blocage (12) pour implants, tels que par exemple des clous médullaires, en particulier des clous médullaires pour tibia, qui peut être vissée dans une paroi de l'os en traversant un ajour ou un évidement de l'implant, la vis de blocage (12) étant réalisée, dans sa zone centrale (15), traversant l'ajour ou l'évidement de l'implant, comme tige non filetée dont le diamètre extérieur (a) correspond sensiblement au contour de l'ajour ou de l'évidement de l'implant et est supérieur ou égal au diamètre extérieur du filetage (16) à l'extrémité libre de la vis de blocage (12), et la vis de blocage (12) présentant, entre la zone centrale (15) et la tête de vis (13), une autre filetage (14) dont le diamètre extérieur est supérieur au diamètre (a) de la zone centrale (15), **caractérisée en ce que** la zone de transition entre la zone centrale (15) non filetée de la vis (12) et l'autre filetage (14), est réalisée comme partie taraudeuse avec des espaces libres pour l'évacuation de la matière taillée, avec une surface auxiliaire tronconique dont les génératrices (21) forment avec l'axe (20) de la vis (12) un angle (β) inférieur à 35°, de préférence inférieur à 25°.

2. Vis de blocage selon la revendication 1, **caractérisée en ce que** l'angle (β) est inférieur à 20°, de préférence environ égal à 15°.

3. Vis de blocage selon la revendication 1 ou 2, **caractérisée en ce que** la zone terminale (17) de la vis (12), située à l'opposé de la tête (13), est réalisée également comme partie taraudeuse dont les génératrices (19) de la surface enveloppante forment avec l'axe (20) un angle (α) inférieur à l'angle compris entre les génératrices (21) et l'axe (20) de la partie taraudeuse venant ensuite dans la direction axiale.

4. Vis de blocage selon la revendication 3, **caractérisée en ce que** l'angle (α) dans la zone terminale (17) située à l'opposé de la tête (13) est inférieur à 25° et de préférence environ égal à 10°.

5. Vis de blocage selon l'une des revendications 1 à 4, **caractérisée en ce que** le diamètre de fond de filet de l'autre filetage (14) correspond au diamètre (a) de la zone centrale (15) non filetée.

6. Vis de blocage selon l'une des revendications 1 à 5, **caractérisée en ce que** les pas des deux parties filetées (14, 16) sont choisis égaux.

7. Vis de blocage selon l'une des revendications 1 à 5, **caractérisée en ce que** le pas de l'autre filetage (14), voisin de la tête de vis (13), est choisi inférieur au pas du filetage (16) à l'extrémité libre de la vis (17).
